Europäisches Patentamt

European Patent Office

Office européen des brevets

(11) Veröffentlichungsnummer: **0 448 760 A1**

## (12) EUROPÄISCHE PATENTANMELDUNG

(21) Anmeldenummer: 90106039.2

(51) Int. Cl.⁵: **H01R 13/639**

(22) Anmeldetag: 29.03.90

(43) Veröffentlichungstag der Anmeldung:
**02.10.91 Patentblatt 91/40**

(84) Benannte Vertragsstaaten:
**DE FR GB IT NL SE**

(71) Anmelder: **Siemens Elema AB**
**Röntgenvägen 2**
**S-171 95 Solna 1(SE)**

(84) **SE**

Anmelder: **Siemens Aktiengesellschaft**
**Wittelsbacherplatz 2**
**W-8000 München 2(DE)**

(84) **DE FR GB IT NL**

(72) Erfinder: **Fröberg, Paul Schwede**
**Virebergsvägen 28**
**S-171 40 Solna(SE)**

(54) **Elektrische Steckverbindung.**

(57) Elektrische Steckverbindung, aufweisend ein erstes und ein zweites Verbindungsteil (3, 4) mit Kontakten (7, 10), die bei zusammengefügter Steckverbindung miteinander in Eingriff stehen. Es sind Mittel zum Sichern der Steckverbindung gegen unbeabsichtigtes Lösen vorgesehen, die eine dem ersten Verbindungsteil (3) zugeordnete Schraubenfeder (17) und einen an dem zweiten Verbindungsteil (4) vorgesehenen Halteabschnitt (16) aufweisen, wobei die Schraubenfeder (17) einen Innendurchmesser aufweist, der um ein solches Maß geringer als der Außendurchmesser des Halteabschnittes (16) ist, daß dieser beim Zusammenfügen der Steckverbindung unter Zusammendrücken der Schraubenfeder (17) und/oder unter Verdrehung der Enden der Schraubenfeder (17) relativ zueinander gegen deren Wicklungssinn in die Schraubenfeder (17) einführbar ist.

FIG 4

Die Erfindung betrifft eine elektrische Steckverbindung, aufweisend ein erstes und ein zweites Verbindungteil mit Kontakten, die bei zusammengefügter Steckverbindung miteinander in Eingriff stehen, und Mittel zum Sichern der Steckverbindung gegen unbeabsichtigtes Lösen.

Bei derartigen Steckverbindungen besteht grundsätzlich das Problem, daß sich die Handhabung der Mittel zum Sichern schwierig gestaltet und der Bauraumbedarf für die Mittel zum Sichern relativ groß ist. So ist z.B. aus der Druckschrift "DIALOG-Schrittmacher 728", Gebrauchsanweisung, Oktober 1986, Siemens-Elema AB, Solna, Schweden, eine Steckverbindung bekannt, die dazu dient, eine endocardielle Elektrode an einen implantier baren Herzschrittmacher anzuschließen. Dabei ist das eine Verbindungteil mit einer Gewindebohrung versehen, in die eine Madenschraube einschraubbar ist, die auf das andere Verbindungsteil einwirkt, um dieses festzuklemmen. Um sicherzustellen, daß einerseits die Madenschraube mit einem für eine sichere Arretierung ausreichenden Drehmoment angezogen wird und andererseits Beschädigungen der Verbindungsteile und der Schraube ausgeschlossen sind, erfolgt das Anziehen der Madenschraube mittels eines sogenannten Allen-Schraubenziehers, der so ausgelegt ist, daß er bei Erreichen des Soll-Drehmomentes bricht. Nach dem Anziehen der Madenschraube wird die Gewindebohrung mittels eines Stopfens aus Silikongummi abgedichtet, um zu verhindern, daß Körperflüssigkeit in das Innere der Steckverbindung eindringen kann. Zum Einführen des Stopfens in die Gewindebohrung ist ein besonderes Werkzeug erforderlich. Es wird deutlich, daß das Zusammenfügen der bekannten Steckverbindung sich äußerst umständlich gestaltet, wobei erschwerend hinzukommt, daß der gesamte Vorgang unter Operationsbedinungen zu erfolgen hat, da der Herzschrittmacher erst dann an die Elektrode angeschlossen ist, wenn diese bereits durch das Venensystem des Patienten zu dessen Herz geführt ist. Auch im Falle von Reoperationen, die beispielsweise erforderlich sein können, um die Elektrodenlage zu korrigieren, eine defekte Elektrode auszutauschen oder einen verbrauchten Herzschrittmacher zu ersetzen, ist die bekannte Steckverbindung schwierig zu handhaben, da es nicht ohne weiteres möglich ist, die Mittel zum Sichern zu deaktivieren, was zum Lösen der Steckverbindung aber erforderlich ist.

Der Erfindung liegt die Aufgabe zugrunde, eine Steckverbindung der eingangs genannten Art so auszubilden, daß die Mittel zum Sichern leicht handhabbar sind und zu keiner wesentlichen Steigerung des Bauraumbedarfes der Steckverbindung führen. Insbesondere soll die Steckverbindung für den Anschluß einer Elektrode an ein implantierbares medizinisches Gerät geeignet sein.

Nach der Erfindung wird diese Aufgabe dadurch gelöst, daß die Mittel zum Sichern eine dem ersten Verbindungteil zugeordnete Schraubenfeder und einen an dem zweiten Verbindungteil vorgesehenen Halteabschnitt aufweisen, wobei die Schraubenfeder einen Innendurchmesser aufweist, der um ein solches Maß geringer als der Außendurchmesser des Halteabschnittes ist, daß der Halteabschnitt beim Zusammenfügen der Steckverbindung unter Verdrehen der Enden der Schraubenfeder relativ zueinander gegen den Wicklungssinn der Schraubenfeder und/oder unter Zusammendrücken der Schraubenfeder in die Schraubenfeder einführbar ist. Die Erfindung macht also von der Eigenschaft von Schraubenfedern Gebrauch, ihren Innendurchmesser zu vergrößern, wenn sie zusammengedrückt und/oder ihre Enden gegen den WicklungsSinn der Schraubenfeder relativ zueinander verdreht werden. Beim Zusammenfügen der erfindungsgemäßen Steckverbindung wird also zunächst eine Vergrößerung des Innendurchmessers der Schraubenfeder bewirkt, die es erlaubt, den Halteabschnitt einzuführen. Sobald die Druckeinwirkung bzw. die Verdrehung der Enden der Schraubenfeder relativ zueinander aufgehoben wird bzw. die Steckverbindung vollständig zusammengefügt ist, übt die Schraubenfeder in ihrem Bestreben, ihren ursprünglichen Innendurchmesser wieder anzunehmen, Normalkräfte auf die mit der Schraubenfeder in Eingriff stehende Oberfläche des Halteabschnittes aus, so daß die Steckverbindung reibschlüssig arretiert ist. Ein Lösen der Steckverbindung ist möglich, wenn die beiden Verbindungteile gegen den Wicklungssinn der Schraubenfeder relativ zueinander verdreht werden und unter gleichzeitiger Zugausübung voneinander getrennt werden. Dabei werden durch die genannte Drehbewegung die von der Schraubenfeder auf die Oberfläche des Halteabschnittes ausgeübten Normalkräfte infolge der mit der Drehbewegung einhergehenden leichten Vergrößerung des Innendurchmessers der Schraubenfeder so weit reduziert, daß die Steckverbindung gelöst werden kann. Es ist leicht einzusehen, daß die erfindungsgemäße Steckverbindung auch unter Operationsbedingungen problemlos zusammengefügt bzw. gelöst werden kann, wobei besonders vorteilhaft ist, daß keinerlei Werkzeuge erforderlich sind, um die Mittel zum Sichern zu aktivieren bzw. zu deaktivieren. Die erfindungsgemäße Steckverbindung ist somit für den Anschluß einer Elektrode an ein implantierbares medizinisches Gerät besonders geeignet. Es kommt hinzu, daß die erfindungsgemäße Steckverbindung nur einen sehr geringen Bauraum beansprucht, was die Verwendung dieser Steckverbindung im Zusammenhang mit implantierbaren medizinischen Geräten zusätzlich begünstigt.

Gemäß einer bevorzugten Variante der Erfindung ist vorgesehen, daß der Halteabschnitt beim Zusammenfügen der Steckverbindung durch Druck und/oder Verdrehen der Verbindungsteile relativ zueinander gegen den Wicklungssinn der Schraubenfeder in die Schraubenfeder einführbar ist. Zum Zusammenfügen der Steckverbindung wird also beispielsweise der Halteabschnitt unter Druck mit der Schraubenfeder in Eingriff gebracht und der Druck erhöht, bis der Halteabschnitt in die Schraubenfeder hineingleitet und so die Kontakte miteinander in Eingriff kommen. Sobald die Druckeinwirkung aufhört, dies ist grundsätzlich dann der Fall, wenn die beiden Verbindungsteile der Steckverbindung vollständig zusammengefügt sind und die Kontakte miteinander in Eingriff sind, legt sich die Schraubenfeder haltend an den Halteabschnitt an. Es besteht auch die Möglichkeit, die Steckverbindung in der Weise zusammenzufügen, daß der Halteabschnitt mit der Schraubenfeder in Eingriff gebracht wird und die beiden Verbindungsteile gegen den Wicklungssinn der Schraubenfeder so weit relativ zueinander verdreht werden, daß der Halteabschnitt praktisch ohne Druckeinwirkung in die Schraubenfeder hineingleitet und die Kontakte miteinander in Eingriff kommen. Hierbei beschränkt sich die Druckausübung auf dasjenige Maß, das erforderlich ist, um diejenigen Reibungskräfte aufbauen zu können, die erforderlich sind, um beim Verdrehen der Verbindungsteile relativ zueinander auch eine Verdrehung der beiden Enden der Schraubenfeder relativ zueinander zu bewirken. Schließlich besteht auch die Möglichkeit, die Steckverbindung zusammenzufügen, indem der Halteabschnitt unter Druckeinwirkung und gleichzeitigem Verdrehen der Verbindungsteile relativ zueinander gegen den Wicklungssinn der Schraubenfeder in die Schraubenfeder eingeführt wird.

Eine besonders vorteilhafte Ausführungsform der Erfindung sieht vor, daß Betätigungsmittel vorgesehen sind, mittels derer die Schraubenfeder derart verformbar ist, daß ihr Innendurchmesser eine Vergrößerung erfährt. Hierdurch ist insbesondere auch eine zusätzliche Erleichterung beim Lösen der Steckverbindung erreicht, da es nun genügt, mittels der Betätigungsmittel eine solche Vergrößerung des Innendurchmessers der Schraubenfeder zu bewirken, daß der Halteabschnitt freigegeben ist und die Verbindungsteile leicht voneinander getrennt werden können. Es besteht jedoch auch die vorteilhafte Möglichkeit, die Betätigungsmittel in entsprechender Weise beim Zusammenfügen der Steckverbindung zu nutzen.

Die Unteransprüche betreffen vorteilhafte Ausführungsformen der Erfindung.

In den beigefügten Zeichnungen sind Ausführungsbeispiele der Erfindung dargestellt. Es zeigen:

Fig. 1    in teilweise geschnittener, schematischer Darstellung einen implantierbaren Herzschrittmacher mit zugehöriger Elektrode, welche eine erfindungsgemäße Steckverbindung aufweisen,

Fig. 2    in stark vergrößerter Darstellung einen Längsschnitt durch den Herzschrittmacher im Bereich des dem Herzschrittmacher zugeordneten Verbindungsteiles der Steckverbindung,

Fig. 3    ebenfalls in stark vergrößerter Darstellung die Elektrode im Bereich des zu der Elektrode gehörigen Verbindungsteiles der Steckverbindung in teilweise geschnittener Darstellung,

Fig. 4    in stark vergrößerter Darstellung einen Längsschnitt durch die Steckverbindung gemäß den Fig. 1 bis 3 in zusammengebautem Zustand,

Fig. 5    eine teilweise Ansicht des Herzschrittmachers in Richtung des Pfeiles Y gemäß Fig. 2, und

Fig. 6    in stark vergrößerter Darstellung einen Längsschnitt durch einen implantierbaren Herzschrittmacher im Bereich des diesem zugeordneten Verbindungsteiles einer zweiten Ausführungsform der erfindungsgemäßen Steckverbindung.

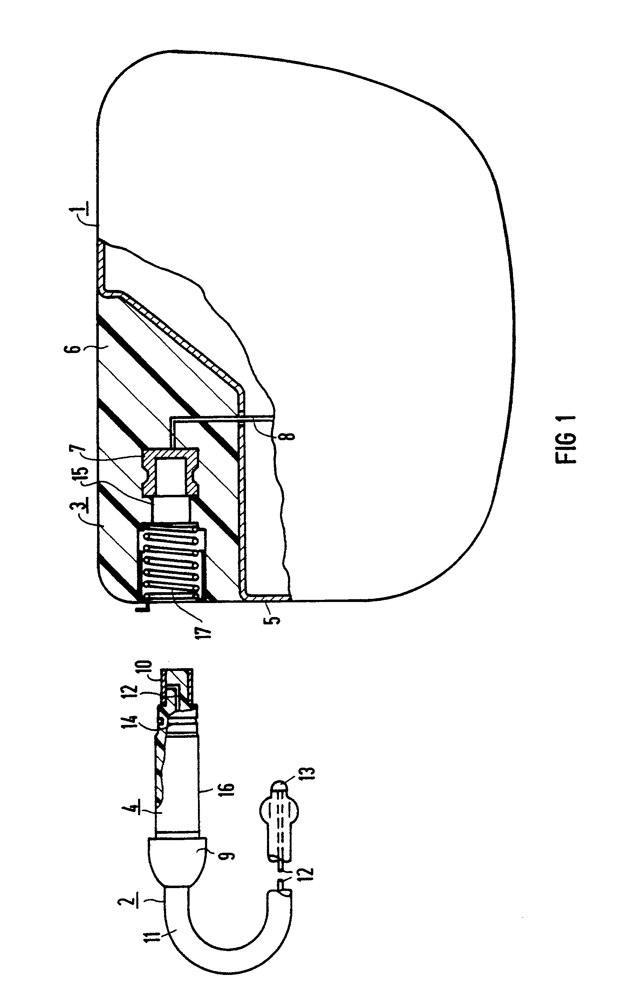

In der Fig. 1 sind ein implantierbarer Herzschrittmacher 1 und eine endokardielle Elektrode 2 dargestellt. Die Verbindung der Elektrode 2 mit dem Herzschrittmacher 1 erfolgt mittels einer Steckverbindung, die ein dem Herzschrittmacher 1 zugeordnetes erstes Verbindungsteil 3 und ein der Elektrode 2 zugeordnetes zweites Verbindungsteil 4 aufweist.

Das erste Verbindungsteil 3 weist einen mit dem beispielsweise aus Titan-Blech gebildeten Gehäuse 5 des Herzschrittmachers 1 fest verbundenen Grundkörper 6 auf, der aus einem elektrisch isolierenden polymeren Werkstoff gebildet ist. In den Grundkörper 6 ist ein etwa napfförmiges Kontaktteil 7, dessen zylindrische Bohrungswand als Kontaktfläche dient, so eingebettet, daß es durch eine in dem ersten Verbindungsteil 3 vorgesehene Bohrung, in die das zweite Verbindungsteil 4 einführbar ist, zugänglich ist. An dem Kontaktteil 7 ist eine elektrische Leitung 8 angebracht, die in das Innere des Gehäuses 5 des Herzschrittmachers 1 führt und mit den dort befindlichen, in Fig. 1 nicht dargestellten elektrischen Komponenten in Verbindung steht.

Das zweite Verbindungsteil 4 weist einen aus einem elektrisch isolierenden polymeren Werkstoff gebildeten, etwa zylindrischen Grundkörper 9 auf, der an seinem freien Ende mit einem etwa hülsenförmigen Kontaktteil 10 versehen ist. Der Grundkörper 9 des zweiten Verbindungsteiles 4 ist an die Isolation des Elektrodenkabels 11 angespritzt, das einen Leiter 12 aufweist, der die Spitze 13 der Elektrode 2 mit dem Kontaktteil 10 elektrisch leitend verbindet. Die Spitze 13 steht bei implantierter Elektrode 2 mit dem zu stimulierenden Herzmuskelgewebe in Verbindung.

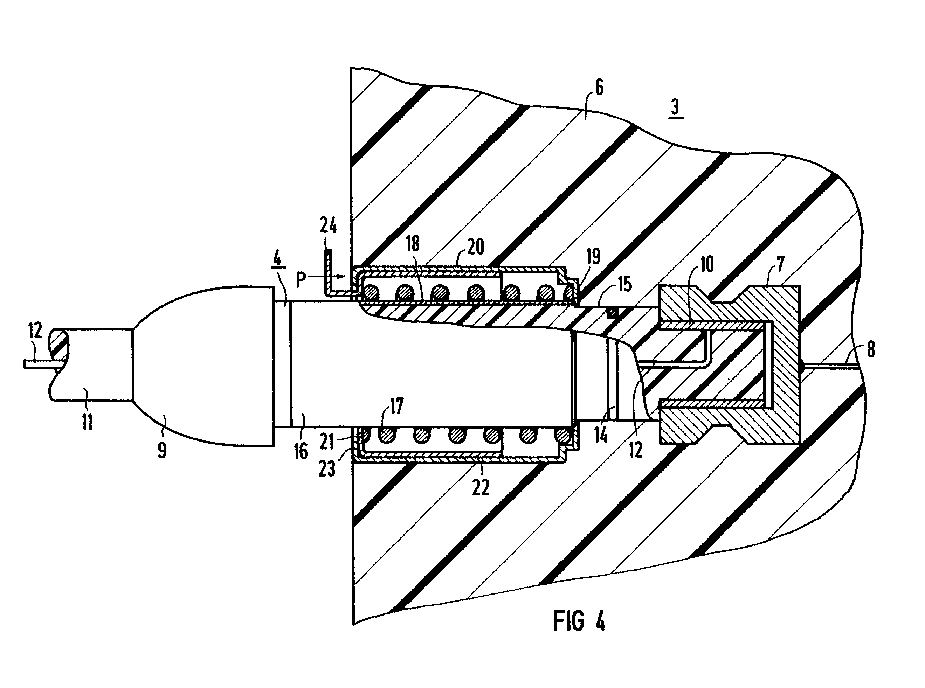

Bei an den Herzschrittmacher 1 angeschlossener Elektrode 2 ist das zweite Verbindungsteil 4 in der aus der Fig. 4 ersichtlichen Weise in die Bohrung des ersten Verbindungsteiles 3 derart eingeführt, daß die äußere Mantelfläche des Kontaktteiles 10 an der Bohrungswandung des Kontaktteiles 7 anliegt, so daß eine elektrisch leitende Verbindung zwischen den elektronischen Bauteilen des Herzschrittmachers 1 und der Spitze 13 der Elektrode 2 besteht. Das zweite Verbindungsteil 4 weist einen neben dem Kontaktteil 10 in einer Nut angeordneten Dichtring 14 auf, der bei zusammengefügter Steckverbindung in der aus Fig. 4 ersichtlichen Weise an einem zylindrischen Abschnitt 15 der Bohrungswand des ersten Verbindungsteiles 3 abdichtend anliegt, so daß jegliche Körperflüssigkeit von den miteinander in Eingriff stehenden Kontaktteilen 7 und 10 ferngehalten ist.

Um ein unbeabsichtigtes Lösen der Steckverbindung zu verhindern, sind Mittel zum Sichern der Steckverbindung vorgesehen, welche zum einen einen an dem zweiten Verbindungsteil 4 vorgesehenen zylindrischen Halteabschnitt 16 und zum anderen eine dem ersten Verbindungsteil 3 zugeordnete, in dessen Bohrung aufgenommene zylindrische Schraubenfeder 17, bei welcher es sich um eine Druckfeder handelt, aufweisen.

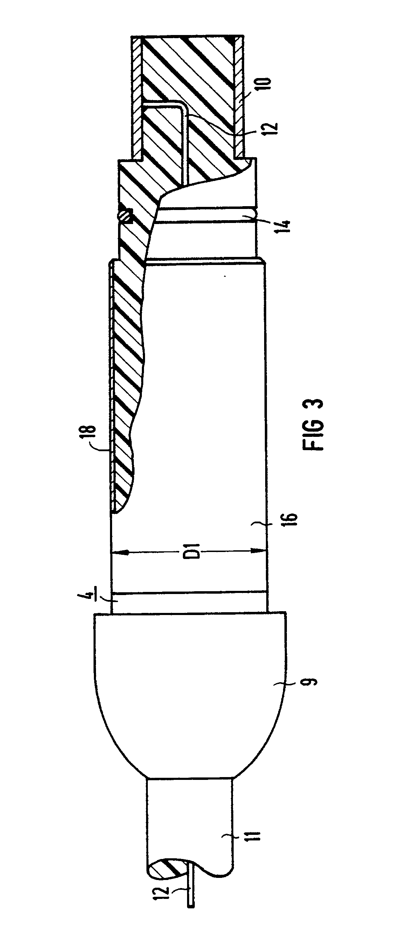

Wie aus der Fig. 3 ersichtlich ist, ist der Halteabschnitt 16 demjenigen Bereich des zweiten Verbindungsteiles 4 benachbart angeordnet, der mit dem Dichtring 14 versehen ist. Im Bereich des Halteabschnittes 16 ist das zweite Verbindungsteil 4 an seiner Mantelfläche mit einer verschleißfesten Beschichtung versehen, bei der es sich beispielsweise, so wie dies in den Fig. dargestellt ist, um eine metallische Hülse 18 handeln kann. Sofern der Grundkörper 9 des zweiten Verbindungsteiles 4 aus einem ausreichend harten und verschleißfesten Werkstoff gebildet ist, kann die Hülse 18 auch entfallen. Der Halteabschnitt 16 weist übrigens den Außendurchmesser D1 auf. Der mit dem Dichtring 14 versehene Bereich des zweiten Verbindungsteiles 4, dessen Halteabschnitt 16 und das Kontaktteil 10 weisen übrigens eine gemeinsame Mittelachse auf.

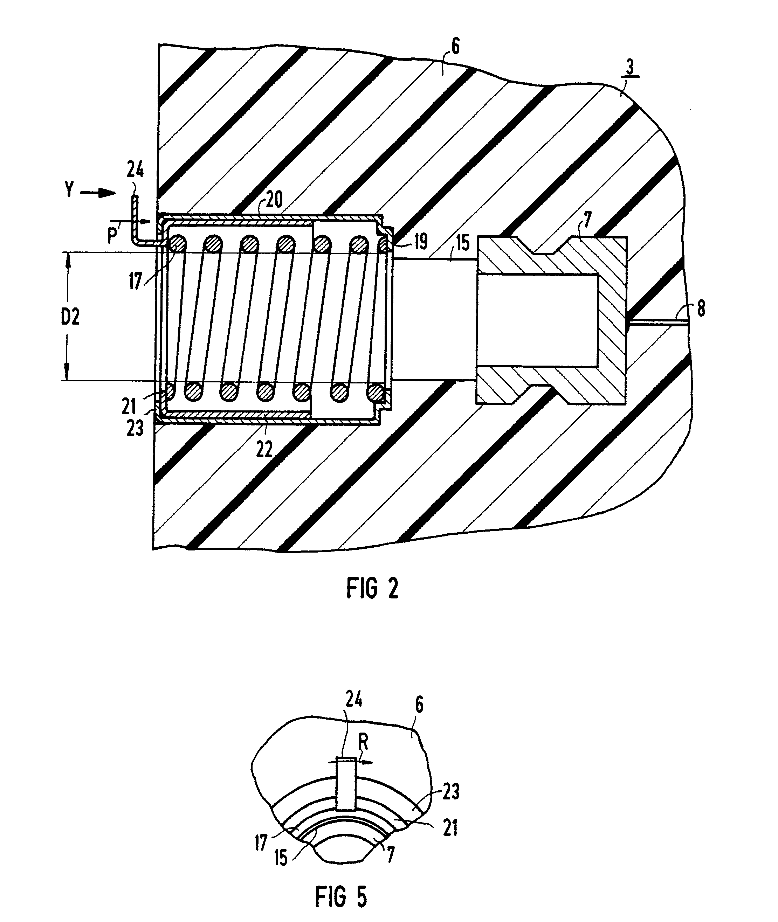

Wie insbesondere aus den Fig. 2 und 4 ersichtlich ist, stützt sich die Schraubenfeder 17 mit ihrem dem Kontaktteil 7 benachbarten Ende gegen den radial einwärts gerichteten Bord 19 einer aus Blech gezogenen Außenhülse 20 ab, während sich das andere Ende der Schraubenfeder 17 gegen den radial einwärts gerichteten Bord 21 einer ebenfalls aus Blech gezogenen Innenhülse 22 abstützt. An ihrem dem Bord 21 der Innenhülse 22 benachbarten Ende weist die Außenhülse 20 einen weiteren radial einwärts gerichteten Bord 23 auf, der an der Außenseite des Bordes 21 der Innenhülse 22 derart anliegt, daß die Schraubenfeder 17 mit einer leichten Vorspannung zwischen den Borden 19 und 21 aufgenommen ist. Die Innenhülse 22 ist in der Außenhülse 20 übrigens im wesentlichen spielfrei aufgenommen, und zwar derart, daß sie relativ zu der Außenhülse 20 verdreht und außerdem axial gegen die Wirkung der Kraft der Schraubenfeder 17 in Richtung auf den Bord 19 der Außenhülse 20 verschoben werden kann. Die aus der Schraubenfeder 17, der Außenhülse 20 und der Innenhülse 22 gebildete Einheit ist in der Bohrung des ersten Verbindungsteiles 3 durch geeignete, nicht dargestellte Maßnahmen, z.B. am Außenumfang der Außenhülse 20 vorgesehene widerhakenartige Krallen, unverdrehbar und axial unverschieblich aufgenommen. Dabei ist die Anordnung derart getroffen, daß die Mittelachsen der Schraubenfeder 17 des Abschnittes 15 der Bohrungswand des ersten Verbindungsteiles 3 und der Bohrungswand des Kontaktteiles 7 zusammenfallen. Die Schraubenfeder 17 weist bei gelöster Steckverbindung gemäß Fig. 2 einen Innendurchmesser D2 auf, der geringfügig, d.h. um ca. 0,1 mm, geringer als der Außendurchmesser D1 des Halteabschnittes 16 ist.

Um die Steckverbindung trotz des Umstandes, daß der Außendurchmesser D1 des Halteabschnittes 16 den Innendurchmesser D2 der Schraubenfeder 17, deren Wicklungssinn dem Uhrzeigersinn entgegengerichtet ist, übersteigt, zusammenfügen zu können, wird von der Eigenschaft von Schraubenfedern Gebrauch gemacht, ihren Innendurchmesser zu vergrößern, wenn sie zusammengedrückt und/oder ihre beiden Enden gegen den Wicklungssinn der Schraubenfeder relativ zueinander verdreht werden. Vorzugsweise wird die erfindungsgemäße Steckverbindung zusammengefügt, indem zunächst das erste Verbindungsteil 4 soweit als möglich, d.h. bis das dem Kontaktteil 10 benachbarte, mit einer Anfasung versehene Ende des Halteabschnittes 16 an der an dem Bord 21 anliegenden Windung der Schraubenfeder 17 anstößt, in das erste Verbindungsteil 3 eingeführt wird. Gegen die Kraft der Schraubenfeder 17 wird nun das zweite Verbindungsteil 4 leicht in das erste Verbindungsteil 3 hineingepreßt und gleichzeitig im Uhrzeigersinn, also gegen den Wicklungssinn der Schraubenfeder 17, relativ zu dem ersten Verbindungsteil 3 verdreht. Dabei wird eine gewisse Vergrößerung des Innendurchmessers der Schraubenfeder 17 dadurch erreicht, daß sie infolge der Druckausübung zusammengepreßt wird. Eine weitere Vergrößerung des Innendurchmessers der Schraubenfeder 17 kommt dadurch zustande, daß infolge von zwischen dem Halteabschnitt 16 und der Schraubenfeder 17 wirkenden Reibungskräften durch das Verdrehen des zweiten Verbindungseiles 4 relativ zu dem ersten Verbindungsteil 3 im Uhrzeigersinn die Enden der Schraubenfeder gegen deren Wicklungssinn relativ zueinander verdreht werden. Wird die Steckverbindung beim Zusammenfügen in der beschriebenen Weise bedient, ergibt sich dasjenige Maß von Druck- und Verdrehung praktisch von selbst, das erforderlich ist, um eine solche Vergrößerung des Innendurchmessers der Schraubenfeder 17 zu erreichen, daß das zweite Verbindungsteil 4 mit seinem Halteabschnitt 16 in die Schraubenfeder 17 so weit hineingleiten kann, so daß die Kontaktteile 7 und 10 in der in Fig. 4 dargestellten Weise vollständig miteinander in Eingriff kommen können. Nach Aufhebung des Druckes und der Verdrehung, eine Druckeinwirkung ist ohnehin dann nicht mehr möglich, wenn die beiden Verbindungsteile der Steckverbindung vollständig zusammengefügt sind, versucht die Schraubenfeder 17 wieder ihren ursprünglichen Innendurchmesser D2 anzunehmen, und legt sich dadurch an den Halteabschnitt 16 an, wodurch die Schraubenfeder 17 Normalkräfte auf die Oberfläche des Halteabschnittes 16 ausübt. Somit ist der Halteabschnitt 16 durch Reibungskräfte in der Schraubenfeder 17 gehaltert und die Steckverbindung gegen unbeabsichtigtes Lösen gesichert.

Außer der beschriebenen Vorgehensweise ist es auch möglich, ausschließlich durch Druck des Halteabschnittes 16 gegen die Schraubenfeder 17 diese so weit zusammenzupressen, daß die zum Zusammenfügen der Steckverbindung erforderliche Vergrößerung des Innendurchmessers der Schraubenfeder 17 eintritt. Weiter ist es möglich, die Steckverbindung zusammenzufügen, indem die beiden Verbindungsteile 3, 4 im wesentlichen ausschließlich relativ zueinander gegen den Wicklungssinn der Schraubenfeder 17 verdreht werden. Dabei beschränkt sich die Druckeinwirkung auf die Schraubenfeder 17 auf dasjenige Maß, das erforderlich ist, um diejenigen Reibungskräfte zwischen dem Halteabschnitt 16 und der Schraubenfeder 17 aufzubauen, die erforderlich sind, um beim Verdrehen der Verbindungsteile 3, 4 relativ zueinander auch eine Verdrehung der beiden Enden der Schraubenfeder 17 relativ zueinander zu bewirken.

Zum Lösen der Steckverbindung wird so vorgegangen, daß die beiden Verbindungsteile 3, 4 relativ zueinander gegen den Wicklungssinn der Schraubenfeder 17 verdreht werden und unter gleichzeitiger Zugausübung voneinander getrennt werden. Dabei werden durch die Drehbewegung die von der Schraubenfeder 17 auf die Oberfläche des Halteabschnittes 16 ausgeübten Normalkräfte infolge der mit der Drehbewegung einhergehenden leichten Vergrößerung des Innendurchmessers der Schraubenfeder 17 so weit reduziert, daß die Steckverbindung getrennt werden kann.

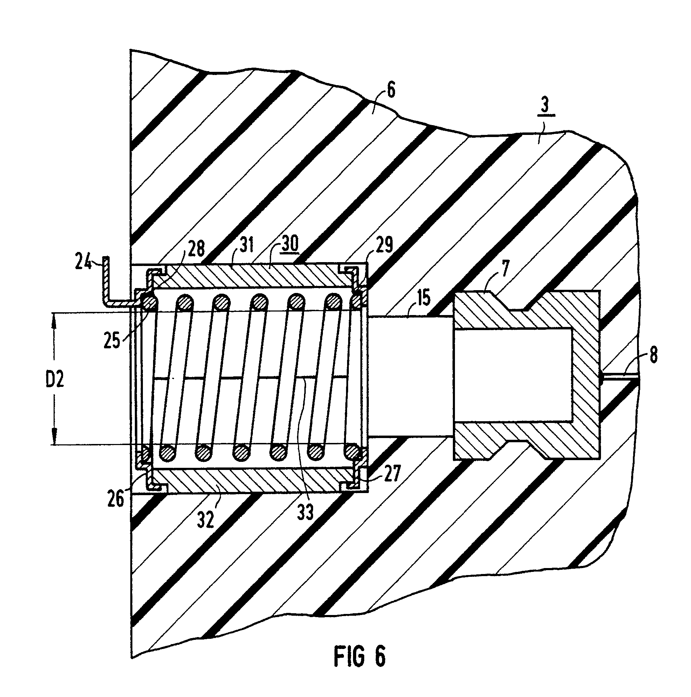

Um das Lösen der Steckverbindung zu erleichtern, ist an den Bord 21 der Innenhülse 22 ein zweifach abgewinkelter Hebel 24 (siehe auch Fig. 5) angebildet. Durch Druckausübung auf den Hebel 24 in Richtung des Pfeiles P und/oder durch Verdrehen des Hebels 24 gegen den Wicklungssinn der Schraubenfeder 17 in Richtung des gekrümmten Pfeiles R (siehe Fig. 5) ist es möglich, durch Zusammenpressen der Schraubenfeder 17 und/oder Verdrehen von deren Enden relativ zueinander gegen den Wicklungssinn eine Vergrößerung des Innendurchmessers der Schraubenfeder 17 zu bewirken, die ausreicht, um die Steckverbindung leicht lösen zu können. Es versteht sich, daß der Hebel 24 in entsprechender Weise auch benutzt werden kann, um die zum Zusammenfügen der Steckverbindung erforderliche Vergrößerung des Innendurchmessers der Schraubenfeder 17 herbeizuführen.

Obwohl in der Regel die zwischen den Enden der Schraubenfeder 17 und den Borden 19, 21 wirkenden Reibungskräfte ausreichend sind, um ein Verdrehen der Enden der Schraubenfeder 17 relativ zueinander, sei es mittels des Hebels 24 oder durch Verdrehen in der beschriebenen Weise der Verbindungsteile 3, 4 relativ zueinander, zu ermöglichen, kann es zweckmäßig sein, eine wenigstens in der Richtung gegen den Wicklungssinn der Schraubenfeder 17 drehfeste Verbindung der Enden der Schraubenfeder 17 mit den Borden 19, 21 oder zumindest des dem Kontaktteil 7 benachbarten Endes des Schraubenfeder 17 mit dem Bord 19 vorzusehen. Derartige drehfeste Verbindungen sind in den Fig. nicht dargestellt. Sie können z.B. durch geeignete Vorsprünge an den Innenseiten der Borde 19 bzw. 21 gebildet sein.

Eine weitere Ausführungsform einer erfindungsgemäßen Steckverbindung ist anhand der Fig. 6 verdeutlicht. Diese zeigt, da deren zweites Verbindungsteil vollständig mit dem der zuvor beschriebenen Steckverbindung übereinstimmt, nur das zweite Verbindungsteil 3. Da auch dieses in vielen Punkten mit dem ersten Verbindungsteil 3 der zuvor beschriebenen Steckverbindung übereinstimmt, sind für jeweils gleiche Teile die gleichen Bezugzeichen verwendet.

Der wesentlichste Unterschied zwischen der Steckverbindung gemäß Fig. 6 und der zuvor beschriebenen besteht darin, daß im Falle der Steckverbindung gemäß Fig. 6 anstelle der als Druckfeder ausgebildeten zylindrischen Schraubenfeder 17 eine als Zugfeder ausgebildete zylindrische Schraubenfeder 25 dem zweiten Verbindungsteil 3 zugeordnet ist. Der Innendurchmesser D2 der Schraubenfeder 25 ist wieder geringfügig geringer als der Außendurchmesser des Halteabschnittes 16 des zweiten Verbindungsteiles 4 (siehe Fig. 3). Mit ihren Enden ist die Schraubenfeder 25 durch Löten mit zwei Blechringen 26, 27 verbunden. Die Lötstellen tragen die Bezugszeichen 28 bzw. 29. Die Blechringe 26, 27 sind mehrfach abgewinkelt, und zwar derart, daß einerseits die Enden der Feder jeweils an einem radial einwärts gerichteten Abschnitt der Blechringe 26, 27 und andererseits axial gerichtete Abschnitte der Blechringe 26 bzw. 27 zentrierend am Außenumfang der Schraubenfeder 25 anliegen. Außerdem besitzen die Blechringe 26 bzw. 27 radial auswärts gerichtete Abschnitte, mit deren Innenseiten sie an den Stirnflächen einer die Schraubenfeder 25 umgebenden Hülse 30 anliegen, sowie axial gerichtete Abschnitte, mittels derer die Hülse 30 zentriert ist. Die Hülse 30 besitzt eine solche Länge, daß die Schraubenfeder 25 leicht vorgespannt ist. Um die Hülse 30 zwischen die Blechringe 26 und 27 montieren zu können, besteht sie aus zwei Halbschalen 31 und 32, eine der Trennfugen ist in Fig. 6 sichtbar und mit 33 bezeichnet, die nacheinander zwischen die Blechringe 26 und 27 gesetzt werden, wenn die Schraubenfeder 25 um das hierzu erforderliche Maß auseinandergezogen wird. Zumindest einer der Blechringe 26, 27 ist relativ zu der Hülse 30 verdrehbar, welche axial unverschieblich und drehfest in der Bohrung des ersten Verbindungsteiles 3 aufgenommen ist.

Da sich eine als Zugfeder ausgebildete Schraubenfeder hinsichtlich ihres Innendurchmessers unter Druckausübung bzw. bei Verdrehen ihrer Enden relativ zueinander gegen den Wicklungssinn bekanntermaßen nicht anders verhält als eine als Druckfeder ausgebildete Schraubenfeder, gelten bezüglich des Zusammenfügens und des Lösens der Steckverbindung gemäß Fig. 6 die im Zusammenhang mit der zuerst beschriebenen Steckverbindung gemachten Ausführungen. Allerdings ist zu beachten, daß infolge der speziellen Konstruktion des ersten Verbindungsteiles 3 gemäß Fig. 6 ein Zusammendrücken der Schraubenfeder 25 ausgeschlossen ist, so daß sowohl das Zusammenfügen als auch das Lösen der Steckverbindung nur in der Weise erfolgen können, daß die erforderliche Durchmesservergrößerung der Schraubenfeder 25 durch Verdrehen ihrer Enden relativ zueinander gegen den Wicklungssinn der Schraubenfeder 25 bewirkt wird.

Im Falle beider Ausführungsbeispiele sind Betätigungsmittel in Form eines Hebels 24 vorhanden. Dieser ist für die Funktion der Steckverbindung jedoch nicht unbedingt erforderlich, sondern fördert lediglich deren leichte Handhabung.

· Die Kontaktierung zwischen den Kontakten 7 und 10 kann in an sich bekannter Weise dadurch gefördert werden, daß wenigstens einer der Kontakte 7 und 10 in nicht dargestellter Weise derart elastisch federnd ausgebildet ist, daß er bei zusammengefügter Steckverbindung an dem jeweils anderen Kontakt federnd anliegt, oder daß nicht dargestellte Kontaktfedern, Kontaktbleche oder dergleichen an den Kontakten 7 und/oder 10 angebracht sind.

Der Kontakt 7 und die Schraubenfeder 17 bzw. 25 im Falle des Verbindungsteiles 3 und der Kontakt 10 und der Halteabschnitt 16 im Falle des zweiten Verbindungsteiles 4 können anders als in den Fig. dargestellt auch so angeordnet sein, daß die Kontakte 7 und 10 in Einsteckrichtung gesehen nicht hinter, sondern vor der Schraubenfeder 17 bzw. 25 und dem Halteabschnitt 16 positioniert sind. Bei einer derart ausgeführten Steckverbindung weisen dann vorzugsweise der Halteabschnitt 16, der mit dem Dichtring 14 versehene Bereich und der Kontakt 10 des zweiten Verbindungsteiles 4 im wesentlichen den gleichen Außendurchmesser auf. Entsprechend weisen die Schraubenfeder 17 bzw. 25, der Bohrungsabschnitt 15 und die Bohrung des Kontaktes 7 des ersten Verbindungsteiles 3 im wesentlichen den gleichen Innendurchmesser auf.

Die Ausführungsbeispiele betreffen ausschließlich die Verwendung der erfindungsgemäßen Steckverbindung im Zusammenhang mit einem implantierbaren medizinischen Gerät. Die Steckverbindung kann jedoch auch für beliebige andere Zwecke verwendet werden.

Bezugszeichenliste

| 1 | Herzschrittmacher |
|---|---|
| 2 | Elektrode |
| 3, 4 | Verbindungteil |
| 5 | Gehäuse |
| 6 | Grundkörper |
| 7 | Kontaktteil |
| 8 | Leitung |
| 9 | Grundkörper |
| 10 | Kontaktteil |
| 11 | Elektrodenkabel |
| 12 | Leiter |
| 13 | Spitze |
| 14 | Dichtring |
| 15 | Abschnitt |
| 16 | Halteabschnitt |
| 17 | Schraubenfeder |
| 18 | Hülse |
| 19 | Bord |
| 20 | Außenhülse |
| 21 | Bord |
| 22 | Innenhülse |
| 23 | Bord |
| 24 | Hebel |
| 25 | Schraubenfeder |
| 26, 27 | Blechring |

| 28, 29 | Lötstelle |
| 30 | Hülse |
| 31, 32 | Halbschale |
| 33 | Trennfuge |
| D1, D2 | Durchmesser |
| P | Pfeil |
| R | gekrümmter Pfeil |

**Patentansprüche**

1. Elektrische Steckverbindung, aufweisend ein erstes und ein zweites Verbindungsteil (3 bzw. 4) mit Kontakten (7 bzw. 10), die bei zusammengefügter Steckverbindung miteinander in Eingriff stehen, und Mittel zum Sichern der Steckverbindung gegen unbeabsichtigtes Lösen, **dadurch gekennzeichnet,** daß die Mittel zum Sichern eine dem ersten Verbindungsteil (3) zugeordnete Schraubenfeder (17; 25) und einen an dem zweiten Verbindungsteil (4) vorgesehenen Halteabschnitt (16) aufweisen, wobei die Schraubenfeder (17; 25) einen Innendurchmesser (D2) aufweist, der um ein solches Maß geringer als der Außendurchmesser (D1) des Halteabschnittes (16) ist, daß der Halteabschnitt (16) beim Zusammenfügen der Steckverbindung unter Verdrehung der Enden der Schraubenfeder (17; 25) relativ zueinander gegen den Wicklungssinn der Schraubenfeder (17; 25) und/oder unter Zusammendrücken der Schraubenfeder (17) in die Schraubenfeder (17; 25) einführbar ist.

2. Steckverbindung nach Anspruch 1, **dadurch gekennzeichnet,** daß der Halteabschnitt (16) beim Zusammenfügen der Steckverbindung durch Druck und/oder durch Verdrehen der Verbindungsteile (3, 4) relativ zueinander gegen den Wicklungssinn der Schraubenfeder (17; 25) in die Schraubenfeder (17; 25) einführbar ist.

3. Steckverbindung nach Anspruch 1 oder 2, **dadurch gekennzeichnet,** daß Betätigungsmittel (24) vorgesehen sind, mittels derer die Schraubenfeder (17; 25) derart verformbar ist, daß ihr Innendurchmesser (D2) eine Vergrößerung erfährt.

4. Steckverbindung nach Anspruch 3, **dadurch gekennzeichnet,** daß die Enden der Schraubenfeder (17; 25) mittels der Betätigungsmittel (24) gegen den Wicklungssinn der Schraubenfeder (17; 25) relativ zueinander verdrehbar sind.

5. Steckverbindung nach Anspruch 3 oder 4, **dadurch gekennzeichnet,** daß die Schraubenfeder (17, 25) mittels der Betätigungsmittel (24) zusammenpreßbar ist.

6. Steckverbindung nach einem der Ansprüche 1 bis 5, **dadurch gekennzeichnet,** daß der Halteabschnitt (16) und zumindest der bei zusammengefügter Steckverbindung mit dem Halteabschnitt (16) in Eingriff stehende Abschnitt der Schraubenfeder (17; 25) wenigstens im wesentlichen zylindrisch ausgebildet sind.

7. Steckverbindung nach einem der Ansprüche 1 bis 6, **gekennzeichnet durch** die Verwendung zum Anschluß einer Elektrode (2) an ein implantierbares medizinisches Gerät.

8. Steckverbindung nach Anspruch 7, **dadurch gekennzeichnet,** daß das zweite Verbindungsteil (4) der Elektrode (2) zugeordnet ist.

9. Steckverbindung nach Anspruch 7 oder 8, **dadurch gekennzeichnet,** daß Dichtmittel (14, 15) vorgesehen sind, welche Körperflüssigkeit von den Kontakten (7, 10) fernhalten.

FIG 1

FIG 2

FIG 5

FIG 3

FIG 4

FIG 6

**Europäisches Patentamt**

# EUROPÄISCHER RECHERCHENBERICHT

Nummer der Anmeldung

## EP 90 10 6039

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 295 872   (R.J. KRAGLE) <br> * Spalte 1, Zeilen 10-22; Spalte 2, Zeilen 31-37; Spalte 3, Zeilen 3-6,21-35; Figuren 1-3 * | 1,3-6 | H 01 R 13/639 |
| Y | | 7-9 | |
| | – – – | | |
| A | EP-A-0 349 791   (WALTER ROSE) <br> * Spalte 3, Zeile 46 - Spalte 4, Zeile 3; Figuren 3-5 * | 1,2,6 | |
| | – – – | | |
| D,Y | "DIALOG-SCHRITTMACHER 728" GEBRAUCHSANWEI-SUNG Oktober 1986, Seiten 4-38, Siemens-Elema AB, Schrittmacher-Abteilung, Solna, Schweden <br> * Seite 34, linke Spalte; Figuren auf Seite 35 * | 7-9 | |
| D,A | idem | 1 | |
| | – – – – – | | |

|   |
|---|
| RECHERCHIERTE SACHGEBIETE (Int. Cl.5) |
| H 01 R 13/00 <br> H 01 R 15/00 <br> H 01 R 9/00 <br> H 01 R 17/00 |

Der vorliegende Recherchenbericht wurde für alle Patentansprüche erstellt

| Recherchenort | Abschlußdatum der Recherche | Prüfer |
|---|---|---|
| Berlin | 14 November 90 | ALEXATOS G |